# FASCICULE DE BREVET EUROPEEN

(11) **EP 2 417 901 B1**
(45) Date de publication et mention de la délivrance du brevet: **25.03.2020**
(21) Numéro de dépôt: 11358007.0
(22) Date de dépôt: 10.08.2011
(51) Int. Cl.: A61B 5/103, A61B 3/11, A61B 5/00

(54) **Procédé et dispositif de contrôle de stimuli**
Verfahren und Vorrichtung zur Kontrolle von Stimuli
Method and device for monitoring stimuli

(30) Priorité: 12.08.2010 FR 1003344
(43) Date de publication de la demande: 15.02.2012
(73) Titulaire: IDMED, 13382 Marseille cedex 13 (FR)
(72) Inventeur: Bernert, Frédéric, 13013 Marseille (FR); Bagnol, Thierry, 13013 Marseille (FR)
(74) Mandataire: Marchand, André

(56) Documents cités:
- EP-A1- 0 236 513
- WO-A1-03/000134
- FR-A1- 2 624 373
- US-A1- 2009 174 865
- RICHARD CHAPMAN C ET AL: "Phasic pupil dilation response to noxious stimulation in normal volunteers: Relationship to brain evoked potentials and pain report", 19990101, vol. 36, no. 1999, 1 janvier 1999 (1999-01-01), pages 44-52, XP007919561, [extrait le 1999-01-01]

## Description

### DOMAINE TECHNIQUE

La présente invention est relative à un procédé de contrôle de stimuli appliqués à un sujet animal ou humain, et à un dispositif de contrôle de ces stimuli.

### ETAT DE LA TECHNIQUE

Il est connu que la pupille d'un sujet animal ou humain change de forme et/ou de taille lorsque le sujet est soumis à une stimulation douloureuse, en particulier une stimulation de type mécanique, électrique, ou thermique.

On constate en particulier que lorsque on fait mal à un sujet, sa pupille se dilate (par réflexe de dilatation pupillaire), que le sujet soit anesthésié ou non. Cette dilatation est inversement proportionnelle à la quantité d'antalgique (tel que la morphine) administrée au sujet.

Les documents HOFLE M. et al, « You can see pain in the eye : Pupillometry as an index of pain intensity under différent luminance conditions », International Journal of Psychophysiology, ELSEVIER, AMSTERDAM, NL, vol. 70, n°3, 1er décembre 2008, pages 171-175, et ELLERMEIER W. et al, « Gender différences in pain ratings and pupil reactions to painful pressure stimuli", PAIN, ELSEVIER SCIENCE PUBLISHERS, AMSTERDAM, NL, vol. 61, 1er janvier 1995, pages 435-439, décrivent des expériences à ce sujet.

Les stimulations peuvent être réalisées par des dispositifs de stimulation thermique algique, des neuro stimulateurs dans le cas de stimulation électrique, des lasers dans le cas de stimulation des récepteurs spécifiques à la douleur.

Les mesures de la pupille peuvent être réalisées par un pupillomètre programmé pour mesurer les variations de taille et de forme de la pupille.

La demande de brevet US2009/0174865 décrit l'utilisation d'un pupillomètre pour tester l'intégrité fonctionnelle d'organes du système nerveux. A cet effet, un microprocesseur du pupillomètre commande, avec un minutage précis, une source de stimuli produisant des jets d'air, des pulvérisations cryogéniques, ou des courants faibles.

Ce document décrit également l'utilisation d'un pupillomètre pour tester la moelle épinière. A cet effet, le pupillomètre commande une vanne délivrant un volume déterminé de CO2 qui est dirigé vers des zones du dermatome pour appliquer des stimuli de froid extrême, et la réponse pupillaire à ces stimuli est évaluée.

Ce document décrit par ailleurs l'utilisation d'un pupillomètre pour tester l'intégrité fonctionnelle d'organes du système auditif. A cet effet, le microprocesseur du pupillomètre commande la fréquence et l'amplitude de stimuli sonores et enregistre la vitesse et l'amplitude de la réponse pupillaire associée à ces stimuli.

L'utilisation d'un tel pupillomètre commandant une source de stimuli pourrait permettre à un opérateur de mesurer la pupille d'un sujet pour chaque stimulation douloureuse qu'on lui applique. Ce procédé aurait l'inconvénient de nécessiter un grand nombre de stimulations douloureuses pour le sujet, pour déterminer un seuil de réactivité de sa pupille à la douleur. Par ailleurs, compte tenu notamment du caractère non linéaire de la réponse pupillaire à des stimuli, les résultats seraient assujettis à la dextérité et à l'évaluation subjective de l'opérateur.

Le document WO03/000134 décrit un dispositif de contrôle de stimuli qui comporte des moyens d'induction de stimuli destinés à être appliqués à la peau d'une personne pour induire des stimuli, et un circuit prévu pour détecter une ouverture de circuit lorsque les moyens d'induction sont détachés de la personne, de façon à enregistrer automatiquement la valeur courante des stimuli.

Le document RICHARD CHAPMAN et al, « Phasic pupil dilatation response to noxious stimulation in normal volunteers : Relationship to brain evoked potentials and pain report », Psychophysiology, Cambridge University Press, USA, vol. 36, 1er janvier 1999, pages 44-52, décrit des procédures de stimulation électrique par des stimuli impulsionnels d'intensité croisssante.

### EXPOSÉ DE L'INVENTION

Un objectif de l'invention est de proposer un procédé de contrôle de stimuli appliqués à un sujet animal ou humain, et un dispositif de contrôle de ces stimuli, qui permette(nt) de déterminer, de façon simple, rapide, peu douloureuse et peu agressive, le niveau de sensibilité à la douleur d'un sujet humain ou animal, via le réflexe de dilatation pupillaire.

Un objectif de l'invention est de proposer un procédé de contrôle de stimuli appliqués à un sujet animal ou humain, et un dispositif de contrôle de ces stimuli, qui soi(en)t amélioré(s) et/ou qui remédie(nt), en partie au moins, aux lacunes ou inconvénients des systèmes connus de contrôle de stimuli.

Un objectif de l'invention est de proposer un procédé de contrôle de stimuli appliqués à un sujet animal ou humain, et un dispositif de contrôle de ces stimuli, qui permette(nt) de stimuler (i.e. faire apparaître) le réflexe de dilatation pupillaire d'un sujet, en soumettant ce sujet à une stimulation douloureuse d'intensité minimale.

Un objectif de l'invention est de proposer un procédé de contrôle de stimuli appliqués à un sujet animal ou humain, et un dispositif de contrôle de ces stimuli, qui permette(nt) de stimuler le réflexe de dilatation pupillaire d'un sujet, en soumettant ce sujet à une stimulation douloureuse calibrée et/ou facilement reproductible.

Selon un aspect de l'invention, il est proposé un pupillomètre intégrant un générateur de stimuli électriques et des moyens de contrôle et d'asservissement des stimuli produits par le générateur, en fonction des modifications de taille et/ou de forme de la pupille qui sont mesurées par le pupillomètre. Le pupillomètre est conforme à la revendication 13.

Selon un autre aspect de l'invention, il est proposé un procédé de contrôle de stimuli appliqués à un sujet animal ou humain, dans lequel on utilise un pupillomètre commandant une source de stimuli électriques, et dans lequel :
- on vérifie l'application correcte des stimuli électriques par le biais d'électrodes de stimulation placées au contact de la peau du sujet ;
- on provoque une augmentation de l'intensité (moyenne) des stimuli électriques, en particulier de la valeur de crête de stimuli impulsionnels, jusqu'à détecter une modification significative de la taille ou de la forme de la pupille, depuis le début de la stimulation ; et
- lorsqu'on détecte une telle modification, on met fin à l'augmentation de l'intensité des stimuli électriques.

Le procédé peut comporter les opérations suivantes :
- maintenir au contact de la peau du sujet deux électrodes servant à appliquer les stimuli électriques ;
- relier les électrodes à un pupillomètre comportant notamment un générateur de stimuli électriques et un module de mesure d'impédance ;
- mesurer l'impédance entre les deux électrodes pour vérifier que leur mise en place est correcte, par comparaison de l'impédance mesurée avec une valeur déterminée d'impédance ;
- appliquer successivement au sujet des stimuli électriques d'intensité croissante délivrés par le générateur, par le biais des électrodes;
- mesurer au moins une caractéristique de taille ou de forme de la pupille d'un œil du sujet, de façon répétée pendant l'application des stimuli électriques,
et, lorsqu'une variation (significative) de la caractéristique mesurée de la pupille est détectée :
- enregistrer au moins une donnée correspondant au(x) stimulus (stimuli) appliqué(s) en dernier lieu ainsi que, généralement, au moins une donnée de sensibilité du sujet à la douleur correspondant à au moins la caractéristique de la pupille mesurée en dernier lieu ; et
- diminuer fortement ou arrêter l'application des stimuli.

Pour détecter une variation significative de la caractéristique mesurée de la pupille, on peut comparer la variation de la caractéristique mesurée à une valeur prédéterminée de variation de cette caractéristique qui est enregistrée dans une mémoire du pupillomètre.

Lorsque la caractéristique mesurée est le diamètre de la pupille, la valeur prédéterminée de variation de cette caractéristique peut être un pourcentage d'augmentation du diamètre.

Ce pourcentage d'augmentation du diamètre est de préférence compris entre 5% et 20%, en particulier voisin de 10%.

Selon des modes de réalisation de ce procédé :
- on peut appliquer successivement au sujet des stimuli d'énergie, de fréquence, ou de durée variable (au cours du temps d'application des stimuli) ;
- on peut appliquer successivement au sujet des stimuli dont l'intensité varie de façon (sensiblement) monotone ;
- on peut appliquer successivement au sujet des stimuli dont l'intensité varie de façon (sensiblement) continue, ou au contraire de façon discontinue ;
- les opérations de mesure de caractéristique(s) de la pupille peuvent comporter des opérations de détermination d'un paramètre choisi parmi une dimension - telle que le diamètre - de la pupille, la surface de la pupille, ou un paramètre de forme de la pupille.

Pour la détermination de la caractéristique de taille ou de forme de la pupille, le procédé comporte généralement des opérations d'éclairage de la pupille par une source de lumière intégrée au pupillomètre, des opérations d'acquisition d'images de la pupille par une caméra du pupillomètre, et des opérations de traitement des images de la pupille délivrées par la caméra, permettant la détermination de la (ou des) caractéristique(s) de la pupille.

Selon un autre aspect de l'invention, il est proposé un dispositif de contrôle de stimuli appliqués à un sujet, en particulier à un être humain, qui est arrangé, en particulier programmé, pour effectuer les opérations d'un procédé selon l'invention.

Selon un autre aspect de l'invention, il est proposé un dispositif de contrôle de stimuli appliqués à un sujet, en particulier à un être humain, qui comporte une paire d'électrodes et un pupillomètre selon la revendication 13 comportant :
- une unité de stimulation reliée à la paire d'électrodes et comportant :
- une unité de commande et de génération de stimuli électriques qui est agencée, en particulier programmée, pour générer successivement des stimuli électriques d'intensité croissante ; et
- une unité de contrôle de l'application des stimuli électriques qui est agencée, en particulier programmée, pour mesurer l'impédance aux bornes des électrodes, i.e. l'impédance du circuit constitué par les électrodes et la partie du corps du sujet sur lequel sont appliquées les électrodes ;
- une unité de mesure agencée, en particulier programmée, pour mesurer au moins une caractéristique de taille ou de forme de la pupille d'un œil du sujet, de façon répétée pendant l'application des stimuli ; et
- une unité de calcul reliée à l'unité de stimulation et à l'unité de mesure et agencée pour, en particulier programmée pour, détecter les variations de la caractéristique mesurée par l'unité de mesure et, lorsqu'une variation (significative) de la caractéristique mesurée est détectée, commander l'arrêt de la croissance ou de l'application des stimuli et enregistrer au moins une donnée correspondant au(x) stimulus (stimuli) appliqué(s) en dernier lieu ainsi que, généralement, au moins la caractéristique mesurée en dernier lieu.

L'unité de mesure du dispositif peut comporter une caméra, un module d'éclairage agencé pour éclairer l'œil du sujet, et un module d'acquisition et de traitement d'images délivrées par la caméra, qui est programmé pour déterminer la caractéristique mesurée de la pupille.

Le dispositif peut comporter une unité de communication reliée à l'unité de calcul de façon à permettre à un utilisateur du dispositif de définir les conditions de mesure, de stimulation, et de contrôle des stimuli.

Selon un autre aspect de l'invention, il est proposé un pupillomètre comportant un dispositif selon l'invention, en particulier un pupillomètre portable.

Un avantage des stimulations électriques est qu'elles sont comparables aux stimulations douloureuses subies lors d'un acte chirurgical. Ainsi, si un sujet à une réponse pupillaire importante à des stimulations électriques, il est probable que l'on ne puisse pas réaliser sur lui un acte chirurgical douloureux (tel qu'une incision sternale) sans que ses paramètres hémodynamiques soient modifiés.

Un autre avantage des stimulations électriques est qu'elles sont facilement reproductibles et ne provoquent pas de lésion.

D'autres aspects, caractéristiques, et avantages de l'invention apparaissent dans la description suivante qui se réfère aux figures annexées et illustre, sans aucun caractère limitatif, des modes préférés de réalisation de l'invention.

### BREVE DESCRIPTION DES FIGURES

La figure 1 illustre l'algorithme de fonctionnement de l'invention
La figure 2 illustre un aspect de l'invention sous la forme d'un schéma fonctionnel du dispositif.
La figure 3 illustre la variation continue de la valeur crête de l'intensité de stimuli appliqués au sujet (axe des ordonnées) en fonction du temps (axe des abscisses).
La figure 4 illustre une variation discontinue de l'intensité moyenne (d'enveloppe) - et de la valeur crête de l'intensité - des stimuli appliqués au sujet (axe des ordonnées) en fonction du temps (axe des abscisses).
La figure 5 illustre une variation discontinue de l'intensité moyenne des stimuli appliqués au sujet (axe des ordonnées de gauche) en fonction du temps (axe des abscisses) avec enregistrement (point A) de la modification significative de taille de la pupille. La taille de la pupille étant représentée par la courbe en pointillés et quantifiée par l'axe des ordonnées de droite.
La figure 6 illustre un aspect détaillé de fonctionnement de la présente invention sous forme d'algorithme.
La figure 7 illustre un pupillomètre portable 10 relié par des fils conducteurs 12 à deux électrodes 11 au contact de l'avant bras d'un sujet humain pour l'application de stimuli électriques produits par le pupillomètre.

### DESCRIPTION DETAILLEE DE L'INVENTION

Sauf indication explicite ou implicite contraire, des éléments ou organes - structurellement ou fonctionnellement - identiques ou similaires sont désignés par des repères identiques sur les différentes figures.

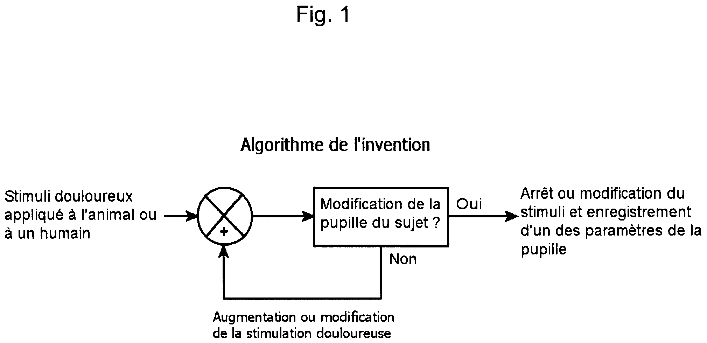

La présente invention a pour objet de contrôler une stimulation douloureuse appliquée à un animal ou un être humain et d'asservir la stimulation aux modifications de sa pupille. Le schéma correspondant à cet asservissement est représenté figure 1.

L'invention permet de tester une éventuelle modification de la pupille d'un sujet à qui on applique une stimulation douloureuse. Elle permet également de déterminer le seuil de sensibilité à la douleur d'une personne ou d'un animal via sa mesure pupillaire, en lui appliquant un minimum de stimulation douloureuse de type électrique pendant un minimum de temps.

Le seuil de la douleur du sujet est déterminé en générant une stimulation douloureuse croissante en puissance jusqu'à mesurer une modification significative de sa pupille.

La détection d'une modification significative de la pupille permet de connaître la valeur de la stimulation électrique minimale ayant permis d'obtenir la modification significative. Cette valeur peut être qualifiée de seuil de sensibilité pupillaire à la douleur du sujet. Ce seuil peut être considéré comme un seuil objectif de la mesure de la douleur perçu par la personne ou l'animal, car il est indépendant du jugement de la personne ou de l'animal soumis à ce test, ainsi que du mode opératoire suivi par l'opérateur.

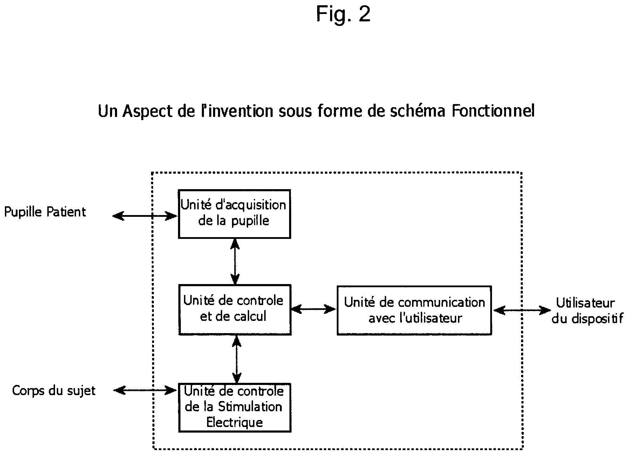

Selon un mode de réalisation, le dispositif de contrôle de stimuli comporte quatre unités ou blocs fonctionnels illustrés figure 2, qui peuvent être incorporés à un pupillomètre portable : une unité d'acquisition qui mesure la pupille, une unité de calcul, une unité de communication avec l'utilisateur, et une unité de stimulation électrique.

L'unité d'acquisition de la pupille a pour fonction d'acquérir des images de la pupille du sujet. A cet effet, on peut éclairer la pupille dans le proche infra rouge notamment, afin de la filmer et d'en extraire des images à une fréquence désirée. L'éclairage de la pupille peut être à bande large, peut couvrir les longueurs d'onde visibles et non visibles, et peut être continu ou intermittent.

Le capteur de l'unité d'acquisition filmant la pupille permet d'obtenir une image de la pupille. Il est de technologie CMOS, CCD, ou autre. Sa sensibilité et sa résolution sont adaptées à la résolution souhaitée pour les mesures de la pupille.

L'unité de calcul est essentiellement constituée d'un microprocesseur, de mémoire associée/reliée au microprocesseur, et des composants nécessaires à son fonctionnement ; l'unité de calcul et/ou le microprocesseur est programmé(e) pour réaliser simultanément les opérations suivantes:
- acquérir les images et les informations sur la pupille envoyées par l'unité d'acquisition,
- calculer la taille, un paramètre de forme, ou d'autre(s) caractéristique(s) de la pupille,
- contrôler, renseigner et interagir avec l'unité interface utilisateur, et
- commander l'unité de stimulation et asservir la génération des stimuli en fonction de paramètres de stimulation enregistrés dans la mémoire, d'une part, et en fonction de la caractéristique mesurée de la pupille, d'autre part.

L'unité d'interface utilisateur permet la communication bidirectionnelle entre l'utilisateur et le dispositif ; elle peut être visuelle, tactile, et/ou sonore. Elle permet à l'utilisateur de définir les conditions de mesure, de stimulation de l'individu, et du contrôle de la stimulation.

Le dispositif, via cette unité d'interface, renseigne l'utilisateur sur les paramètres de stimulation et de mesure du pupillomètre, ainsi que sur les résultats des mesures.

Les résultats affichés à l'utilisateur peuvent être:
_ un tableau contenant les valeurs (en pourcentage) de variation de la pupille pour chaque valeur de stimulation appliquée au sujet ;
_ la valeur de l'intensité de la stimulation à l'instant où a été détectée la variation significative du diamètre de la pupille (par exemple 10% de dilatation pupillaire) ; et
- la durée écoulée entre le début de la séquence de stimulation et de mesure, et l'instant où a été détectée cette variation significative.

L'unité d'interface peut permettre au praticien de stopper en temps réel la génération des stimuli si la réponse de la pupille devient trop importante.

Cette unité d'interface est incorporée ou non au pupillomètre ; dans le cas où elle est externe au pupillomètre, la communication avec ce dernier peut être filaire, par fibre optique, ou par liaison radio. Cette fonction peut être assurée par un dispositif externe renseignant l'utilisateur sur le fonctionnement et sur les paramètres médicaux du sujet.

L'unité de stimulation a pour fonction de stimuler le sujet dont on veut évaluer la réactivité pupillaire à la douleur. L'unité de stimulation est contrôlée par l'unité de calcul du dispositif.

La forme et la puissance de la stimulation peuvent être variées et les variations (au cours d'un cycle de mesure) de la stimulation peuvent être continues ou discontinues. L'intérêt d'une stimulation électrique croissante est d'augmenter progressivement - et de permettre de limiter - la sensation douloureuse perçue par la personne ou l'animal à qui on l'applique.

La bande de puissance couverte ainsi que la puissance maximale de la stimulation peuvent être paramétrables par l'utilisateur afin de pouvoir stimuler des personnes ayant des seuils de sensibilité à la douleur normaux ou faussés (par une anesthésie par exemple).

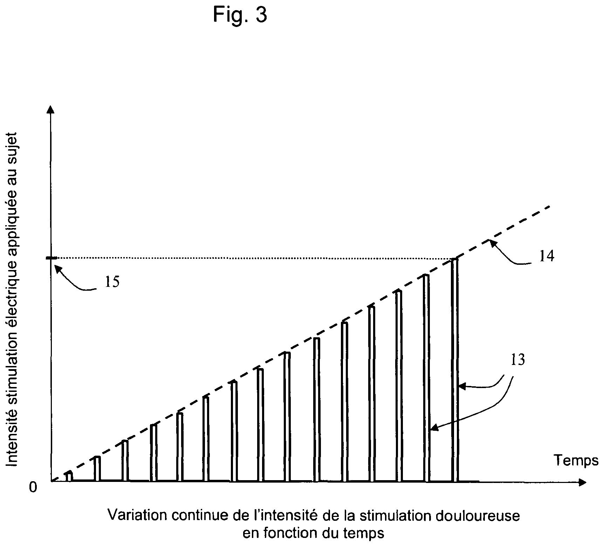

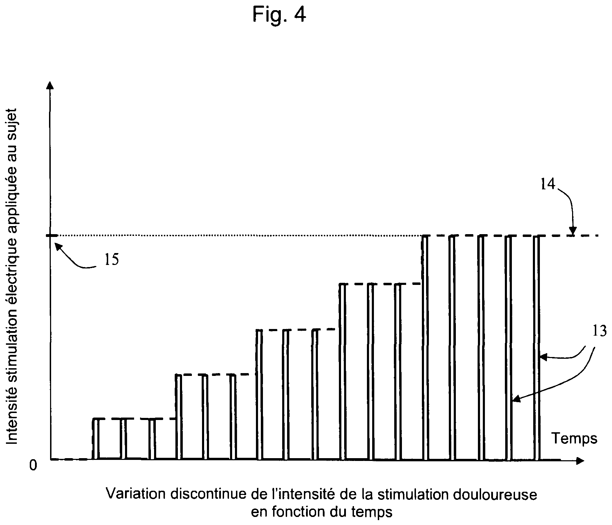

Lorsque les stimuli électriques sont des impulsions 13 électriques, l'enveloppe 14 des variations de la valeur crête de l'intensité des stimuli au cours du temps suit une courbe globalement ascendante, ce qui correspond à une intensité moyenne croissante, comme illustré par les figures 3 et 4.

La figure 3 montre un profil de stimulation continue où la puissance de la stimulation électrique douloureuse est linéairement croissante en fonction du temps, à partir d'une valeur nulle.

La figure 4 montre un profil de stimulation électrique croissante discontinue (par paliers), également à partir d'une valeur nulle.

D'autres modes de réalisation sont possibles, notamment en contrôlant l'intensité des impulsions électriques afin qu'elles s'inscrivent dans une courbe enveloppe en forme de courbe exponentielle ou logarithmique croissant continument ou par paliers.

Les courbes enveloppe sont généralement enregistrées dans une mémoire associée au microprocesseur du pupillomètre.

La croissance de la valeur crête de l'intensité des stimuli s'opère généralement entre une valeur initiale d'intensité est nulle (comme illustré figures 3 et 4), et une valeur finale 15 d'intensité qui est une valeur d'intensité maximale appliquée.

Ces valeurs initiale et finale d'intensité, ainsi que la forme de la courbe de croissance de l'intensité, peuvent être des paramètres prédéterminés qui sont enregistrés dans une mémoire du pupillomètre, et qui sont le cas échéant ajustables par l'opérateur.

Pour ne pas être nuisible au sujet, la valeur maximale et finale 15 d'intensité est de préférence inférieure à - ou voisine de - 100 milliampère (mA), par exemple voisine de 50 mA à 100 mA.

Selon un mode préféré de réalisation, le rythme moyen de croissance de l'intensité des stimuli impulsionnels au cours d'un cycle de mesure, est inférieur ou égal à 50 milliampère par seconde (mA/s) environ, en particulier situé dans une plage allant de 0.1 mA/s environ à 50 mA/s environ, notamment voisin de 2 mA/s environ à 10 mA/s environ.

Il est cependant avantageux que ce taux moyen de croissance de l'intensité des stimuli soit suffisamment élevé pour qu'une réaction pupillaire significative intervienne dans un laps de temps réduit, par exemple dans les secondes qui suivent le démarrage de l'application des stimuli.

Dans le cas d'une stimulation croissant par paliers come illustré figure 4, la durée de chaque palier est de préférence au moins égale à 200 millisecondes.

Le nombre de paliers est de préférence inférieur à 100 environ, en particulier de l'ordre d'une dizaine, ou moins, ce qui facilite leur mémorisation par un opérateur.

A titre d'exemple, pour une stimulation par paliers, on peut prévoir une courbe enveloppe des stimuli comportant cinq paliers d'une durée identique voisine d'une seconde, dont les valeurs croissent par pas de 10 mA ; les intensités successives de stimulation sont alors de zéro, 10, 20, 30, 40, et 50 mA, le taux moyen de croissance de l'intensité étant alors de 10 mA/s.

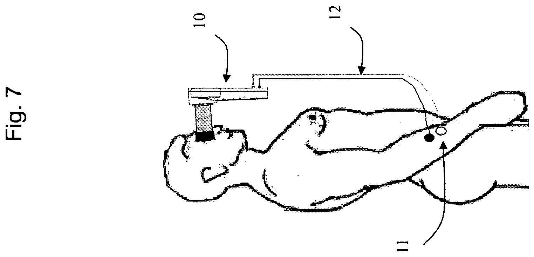

L'utilisation du dispositif est la suivante : le pupillomètre 10 est appliqué sur le visage du sujet afin de filmer un de ses yeux, et deux électrodes 11 de stimulation électrique sont positionnées sur le corps du sujet (cf. figure 7).

La paupière de l'œil filmé par le dispositif est maintenue ouverte. Les électrodes sont positionnées sur la peau du sujet, généralement à proximité d'une terminaison nerveuse, par exemple à proximité d'un des deux nerfs cubitaux.

Le dispositif vérifie dans un premier temps que la mesure de la pupille est satisfaisante et que la connexion des électrodes au sujet est correcte, par une mesure de l'impédance aux bornes des électrodes.

Lorsque ces vérifications sont faites et donnent un résultat positif, l'unité de communication du dispositif signale à l'utilisateur la possibilité de démarrer le processus de mesure ; alternativement, le microprocesseur peut commander un démarrage automatique d'une séquence de mesures.

Le dispositif mesure la taille et la forme de la pupille avec une fréquence d'acquisition et de mesure suffisamment élevée, et commande simultanément la génération de la stimulation électrique.

Le contrôle de la stimulation permet d'en augmenter la puissance en fonction des modifications de la taille et de forme de la pupille. Dès qu'une modification significative mesurable de la pupille est enregistrée par le dispositif, il stoppe la croissance de la stimulation électrique et enregistre les caractéristiques de stimulation et de mesure de la pupille.

Selon un mode de réalisation, l'appareil commande la génération de stimuli selon un profil de stimulation déterminé tel que ceux décrits ci avant, soit jusqu'à la fin de sa durée programmée (i.e. jusqu'à atteindre l'intensité maximale/finale), soit jusqu'à une interruption de cette génération à un instant donné, et commande alors la mémorisation des caractéristiques de la stimulation et des modifications de taille de la pupille.

L'interruption par le pupillomètre, de façon dynamique, de la stimulation, peut être déclenchée lorsque le daimètre de la pupille a varié, depuis le début de la stimulation, de plus de 10%. D'autres valeurs de la variation significative de la caractéristique mesurée peuvent être utilisées, par exemple 20% notamment lorsque la caractéristique mesurée est la surface de la pupille.

Notamment lorsque la caractéristique mesurée est le diamètre de pupille, on cherche généralement à ne pas dépasser une valeur pour laquelle on constate des modifications des marqueurs de la douleur chez le sujet, tels que la fréquence cardiaque, la pression artérielle, ou la sudation.

Cette valeur (en pourcentage) de la variation significative de la caractéristique mesurée, est fonction de l'amplitude et de la vitesse de variation de la stimulation, ainsi que du temps de réponse de la pupille.

Ainsi, le dispositif détermine de façon automatique la puissance de la stimulation électrique douloureuse à laquelle le sujet est réactif via la modification de taille ou de forme de sa pupille.

La fréquence d'acquisition d'images de la pupille et la fréquence d'analyse de ces images pour déterminer le diamètre - ou un autre paramètre de taille ou de forme - de la pupille, sont adaptées à la dynamique de la pupille ; ces fréquences sont généralement au moins égales à une dizaine de Hertz (Hz), en particulier de l'ordre de 50 Hz environ à 100 Hz environ, ou plus.

Lorsque les stimuli électriques sont des impulsions, ils sont généralement générés sous la forme d'un signal périodique (en forme de carré ou créneau) dont la fréquence est également au moins égale à une dizaine de Hertz, en particulier au moins égale à la fréquence d'acquisition d'images de la pupille.

Chaque stimulus peut être une impulsion dont la durée est bien inférieure à la période de ce signal périodique, étant par exemple de l'ordre du dixième ou du centième de cette période. Par exemple, pour des stimulis impulsionnels générés à une fréquence de 100 Hz, la durée de chaque stimulus peut être de l'ordre de 200 micro seconde.

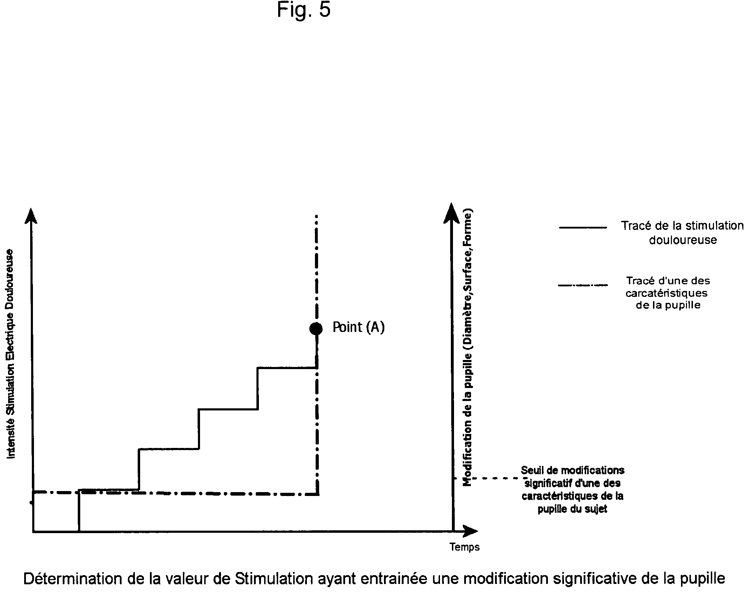

Le point A inscrit sur la figure 5 représente le point de sensibilité pupillaire du sujet à un niveau de stimulation électrique douloureuse.

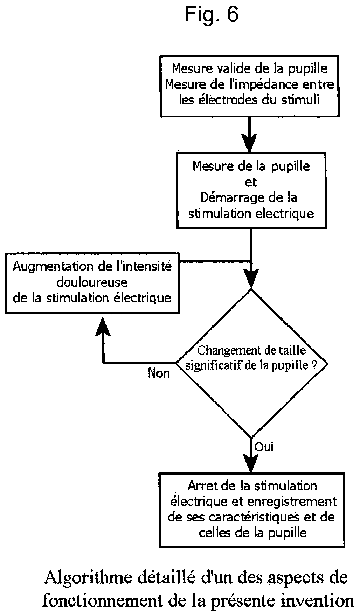

La figure 6 montre l'algorithme détaillé de fonctionnement de la présente invention permettant la détermination du niveau de stimulation douloureuse entraînant une modification de taille de la pupille.

Dans le cas ou la puissance maximale est atteinte sans qu'une modification significative de la pupille n'ait été détectée, le sujet peut être considéré comme non réactif aux stimulations générées par le dispositif.

Dans ce cas, l'unité de stimulation peut, comme illustré figure 4, commander le maintien de la stimulation à sa valeur maximale/finale, pendant une durée réduite qui est par exemple égale à celle d'un des paliers, afin de vérifier la réactivité de la pupille.

## Revendications

1. Procédé de contrôle de stimuli appliqués à un sujet animal ou humain, dans lequel on utilise un pupillomètre comportant une unité de génération de stimuli électriques, qui comporte les opérations suivantes :
- maintenir au contact de la peau du sujet deux électrodes servant à appliquer des stimuli électriques ;
- relier les électrodes au pupillomètre comportant une unité de mesure agencée pour mesurer au moins une caractéristique de taille ou de forme de la pupille d'un œil du sujet, et comportant un module de mesure d'impédance et une unité de calcul;
- mesurer, par le module de mesure d'impédance, l'impédance entre les deux électrodes pour vérifier que leur mise en place est correcte, par comparaison de l'impédance mesurée avec une valeur déterminée d'impédance ;
- appliquer successivement au sujet des stimuli électriques délivrés par l'unité de génération dont l'intensité est croissante entre une valeur initiale et une valeur finale qui sont enregistrées dans une mémoire du pupillomètre, par le biais des électrodes;
- mesurer, par l'unité de mesure, au moins une caractéristique de taille ou de forme de la pupille d'un œil du sujet, de façon répétée pendant l'application des stimuli électriques,
et, lorsqu'une variation significative de la caractéristique mesurée de la pupille est détectée par l'unité de calcul, enregistrer au moins une donnée correspondant aux stimuli appliqués au sujet, et diminuer ou arrêter l'application des stimuli,
et dans lequel, pour détecter une variation significative de la caractéristique mesurée de la pupille, on compare la variation de la caractéristique mesurée à une valeur prédéterminée de variation de cette caractéristique qui est enregistrée dans une mémoire du pupillomètre.

2. Procédé selon la revendication 1 dans lequel, la caractéristique mesurée étant le diamètre de la pupille, la valeur prédéterminée de variation de cette caractéristique est un pourcentage d'augmentation du diamètre de la pupille.

3. Procédé selon la revendication 2 dans lequel le pourcentage d'augmentation du diamètre est compris entre 5 (%) et 20 (%), en particulier voisin de 10 (%).

4. Procédé selon l'une quelconque des revendications 1 à 3 dans lequel on applique successivement au sujet des stimuli dont l'intensité varie de façon continue.

5. Procédé selon l'une quelconque des revendications 1 à 4 dans lequel on applique successivement au sujet des stimuli dont l'intensité varie de façon discontinue.

6. Procédé selon l'une quelconque des revendications 1 à 5 dans lequel les opérations de mesure de caractéristique(s) de la pupille comportent des opérations de détermination d'un paramètre choisi parmi une dimension - telle que le diamètre - de la pupille, la surface de la pupille, ou un paramètre de forme de la pupille.

7. Procédé selon l'une quelconque des revendications 1 à 6 qui comporte des opérations d'éclairage de la pupille par une source de lumière intégrée au pupillomètre, des opérations d'acquisition d'images de la pupille par une caméra du pupillomètre, et des opérations de traitement des images de la pupille délivrées par la caméra, permettant la détermination de la caractéristique de la pupille.

8. Procédé selon l'une quelconque des revendications 1 à 7 dans lequel la stimulation croît par paliers, le nombre total de paliers d'une séquence de stimulation étant de préférence inférieur à 100.

9. Procédé selon l'une quelconque des revendications 1 à 8 dans lequel le rythme moyen de croissance de la stimulation est situé dans une plage allant de 0.1 milliampère par seconde environ à 50 milliampère par seconde environ.

10. Procédé selon l'une quelconque des revendications 1 à 9 dans lequel les stimuli sont des impulsions formant un signal dont la fréquence est supérieure à 10 Hz.

11. Dispositif de contrôle de stimuli appliqués à un sujet animal ou humain, qui comporte une paire d'électrodes et un pupillomètre comportant :
- une unité de stimulation reliée à la paire d'électrodes et comportant :
- une unité de génération de stimuli électriques qui est agencée, en particulier programmée, pour générer successivement des stimuli électriques d'intensité croissante entre une valeur initiale et une valeur finale qui sont enregistrées dans une mémoire du pupillomètre; et
- une unité de contrôle de l'application des stimuli électriques qui est agencée, en particulier programmée, pour mesurer l'impédance aux bornes des électrodes;
- une unité de mesure agencée, en particulier programmée, pour mesurer au moins une caractéristique de taille ou de forme de la pupille d'un œil du sujet, de façon répétée pendant l'application des stimuli ; et
- une unité de calcul reliée à l'unité de stimulation et à l'unité de mesure et agencée, en particulier programmée, pour détecter les variations de la caractéristique mesurée par l'unité de mesure et pour, lorsqu'une variation significative de la caractéristique mesurée est détectée, commander l'arrêt de la croissance ou de l'application des stimuli et enregistrer au moins une donnée correspondant aux stimuli appliqués en dernier lieu ainsi que, généralement, au moins la caractéristique mesurée en dernier lieu,
et dans lequel, pour détecter une variation significative de la caractéristique mesurée de la pupille, l'unité de calcul compare la variation de la caractéristique mesurée à une valeur prédéterminée de variation de cette caractéristique qui est enregistrée dans une mémoire du pupillomètre.

12. Dispositif selon la revendication 11 qui comporte une caméra, un module d'éclairage agencé pour éclairer l'œil du sujet, un module d'acquisition et de traitement d'images délivrées par la caméra et permettant de déterminer la caractéristique de la pupille, et une unité de communication reliée à l'unité de calcul de façon à permettre à un utilisateur du dispositif de définir les conditions de mesure, de stimulation, et de contrôle des stimuli, ledit dispositif étant arrangé, en particulier programmé, pour effectuer des opérations d'un procédé selon l'une quelconque des revendications 1 à 10.

## Patentansprüche

1. Verfahren zur Kontrolle von an ein tierisches oder menschliches Subjekt abgegebenen Stimuli, bei dem ein Pupillometer verwendet wird, das eine elektrische Stimulus-Erzeugungseinheit umfasst, wobei das Verfahren folgende Schritte umfasst:
- Anbringung von zwei Elektroden auf der Haut des Subjekts, damit diese elektrische Stimuli abgeben können;
- Verbindung der Elektroden mit dem Pupillometer, welches eine Messeinheit umfasst, die so angeordnet ist, dass sie mindestens einen Größen- oder Form-Kennwert der Pupille eines Auges des Subjekts misst, und ein Impedanzmessmodul und eine Recheneinheit umfasst;
- Messung der Impedanz zwischen den beiden Elektroden mit dem Impedanzmessmodul, um die korrekte Positionierung der Elektroden durch Vergleich der gemessenen Impedanz mit einem vorbestimmten Impedanzwert zu überprüfen;
- mithilfe der Elektroden sukzessive Abgabe an das Subjekt von elektrischen Stimuli, die von der Erzeugungseinheit geliefert werden und deren Intensität zwischen einem Anfangswert und einem Endwert, welche in einem Speicher des Pupillometers gespeichert sind, zunimmt;
- mit der Messeinheit mehrfache Messung mindestens eines Größen- oder Form-Kennwerts der Pupille eines Auges des Subjekts während der Abgabe elektrischer Stimuli,
und, wenn die Recheneinheit eine signifikante Veränderung des gemessenen Pupillenkennwerts erkennt, Aufzeichnung mindestens eines Datenwerts, der den an das Subjekt abgegebenen Stimuli entspricht, und Verminderung oder Beendigung der Stimuliabgabe,
und bei dem, um eine signifikante Änderung des gemessenen Kennwerts der Pupille zu erkennen, die Änderung des gemessenen Kennwerts mit einem vorbestimmten Änderungswert dieses Kennwerts verglichen wird, der in einem Speicher des Pupillometers aufgezeichnet ist.

2. Verfahren nach Anspruch 1, bei dem, wenn der gemessene Kennwert der Pupillendurchmesser ist, der vorbestimmte Änderungswert dieses Kennwerts eine prozentuale Zunahme des Pupillendurchmessers ist.

3. Verfahren nach Anspruch 2, bei dem die prozentuale Zunahme des Durchmessers zwischen 5 (%) und 20 (%), insbesondere bei etwa 10 (%) liegt.

4. Verfahren nach einem der Ansprüche 1 bis 3, bei dem an das Subjekt sukzessiv Stimuli abgegeben werden, deren Intensität sich kontinuierlich ändert.

5. Verfahren nach einem der Ansprüche 1 bis 4, bei dem an das Subjekt sukzessiv Stimuli abgegeben werden, deren Intensität sich diskontinuierlich ändert.

6. Verfahren nach einem der Ansprüche 1 bis 5, bei dem die Schritte zur Messung des/der Pupillenkennwert(s)/(e) Schritte zur Bestimmung eines Parameters umfassen, der aus einem Größenmaß wie Pupillendurchmesser, Pupillenfläche oder einem Parameter der Pupillenform ausgewählt wird.

7. Verfahren nach einem der Ansprüche 1 bis 6, welches Schritte zur Beleuchtung der Pupille durch eine in das Pupillometer integrierte Lichtquelle, Schritte zur Aufnahme von Bildern der Pupille durch eine Kamera des Pupillometers und Schritte zur Verarbeitung der von der Kamera gelieferten Bilder der Pupille umfasst, wodurch der Pupillenkennwert bestimmt werden kann.

8. Verfahren nach einem der Ansprüche 1 bis 7, bei dem die Stimulation stufenweise ansteigt, wobei die Gesamtzahl der Stufen in einer Stimulationssequenz vorzugsweise weniger als 100 beträgt.

9. Verfahren nach einem der Ansprüche 1 bis 8, bei dem die durchschnittliche Anstiegsrate der Stimulation in einem Bereich von etwa 0,1 Milliampere pro Sekunde bis etwa 50 Milliampere pro Sekunde liegt.

10. Verfahren nach einem der Ansprüche 1 bis 9, bei dem die Stimuli Impulse sind, die ein Signal mit einer Frequenz von mehr als 10 Hz bilden.

11. Vorrichtung zur Kontrolle von an ein tierisches oder menschliches Subjekt abgegebenen Stimuli, die ein Elektrodenpaar und ein Pupillometer umfasst, mit:
- einer Stimulationseinheit, die mit dem Elektrodenpaar verbunden ist und umfasst:
- eine elektrische Stimulus-Erzeugungseinheit, die so angeordnet und insbesondere programmiert ist, dass sukzessiv elektrische Stimuli von zunehmender Intensität zwischen einem Anfangswert und einem Endwert erzeugt werden, die in einem Speicher des Pupillometers aufgezeichnet sind; und
- eine Einheit zur Kontrolle der Abgabe von elektrischen Stimuli, die so angeordnet und insbesondere programmiert ist, dass die Impedanz an den Elektrodenanschlüssen gemessen wird;
- einer Messeinheit, die so angeordnet und insbesondere programmiert ist, dass mindestens ein Größen- oder Form-Kennwert der Pupille eines Auges des Subjekts während der Abgabe der Stimuli mehrfach gemessen wird; und
- einer Recheneinheit, die mit der Stimulationseinheit und mit der Messeinheit verbunden ist und die so angeordnet und insbesondere programmiert ist, dass sie Veränderungen des von der Messeinheit gemessenen Kennwerts erkennt und, wenn eine signifikante Veränderung des gemessenen Kennwerts erkannt wird, die Beendigung des Anstiegs oder der Abgabe der Stimuli steuert und mindestens einen Datenwert aufzeichnet, der den zuletzt abgegebenen Stimuli und allgemein mindestens dem zuletzt gemessenen Kennwert entspricht,
und bei der, um eine signifikante Änderung des gemessenen Kennwerts der Pupille zu erkennen, die Recheneinheit die Änderung des gemessenen Kennwerts mit einem vorbestimmten Änderungswert dieses Kennwerts vergleicht, der in einem Speicher des Pupillometers aufgezeichnet ist.

12. Vorrichtung nach Anspruch 11, die eine Kamera, ein Beleuchtungsmodul, das so angeordnet ist, dass es das Auge des Subjekts beleuchtet, ein Modul zur Erfassung und Verarbeitung von Bildern, die von der Kamera geliefert werden und es ermöglichen, den Kennwert der Pupille zu bestimmen, und eine Kommunikationseinheit umfasst, die mit der Recheneinheit verbunden ist, um es einem Benutzer der Vorrichtung zu ermöglichen, die Bedingungen für die Messung, die Stimulation und die Kontrolle der Stimuli zu definieren, wobei die Vorrichtung so angeordnet und insbesondere programmiert ist, dass sie Schritte eines Verfahrens nach einem der Ansprüche 1 bis 10 ausführt.

## Claims

1. A method of controlling stimuli applied to an animal or human subject, in which use is made of pupillometer including a generator unit for generating electrical stimuli, the method comprising the following operations:
• keeping two electrodes for applying electrical stimuli in contact with the skin of the subject;
• connecting the electrodes to the pupillometer including a measurement unit arranged to measure at least one size or shape characteristic of the pupil of an eye of the subject, and including an impedance-measuring module and a calculation unit;
• using the impedance-measuring module to measure the impedance between the two electrodes in order to verify that they are correctly positioned, by comparing the measured impedance with a determined impedance value;
• using the electrodes to apply to the subject successive electrical stimuli as delivered by the generator unit, the stimuli being of intensity that increases between an initial value and a final value, which values are stored in a memory of the pupillometer;
• using the measurement unit in repeated manner while applying the electrical stimuli in order to measure at least one size or shape characteristic of the pupil of an eye of the subject; and
• when the calculation unit detects a significant variation in the measured characteristics of the pupil, storing data corresponding to at least one stimulus applied to the subject, and decreasing or stopping application of the stimuli; and
wherein, in order to detect a significant variation of the measured characteristic of the pupil, the variation of the measured characteristic is compared with a predetermined value for variation of this characteristic, which value is stored in a memory of the pupillometer.

2. A method according to claim 1, wherein the measured characteristic is the diameter of the pupil, and the predetermined value for variation of this characteristic is a percentage increase in the diameter of the pupil.

3. A method according to claim 2, wherein the percentage increase in the diameter lies in the range of 5% to 20%, and in particular is close to 10%.

4. A method according to any one of claims 1 to 3, wherein the successive stimuli applied to the subject are of intensity that varies in continuous manner.

5. A method according to any one of claims 1 to 4, wherein the successive stimuli applied to the subject are of intensity that varies in discontinuous manner.

6. A method according to any one of claims 1 to 5, wherein the operations of measuring one or more characteristics of the pupil include operations of determining a parameter selected from a dimension such as the diameter of the pupil, the area of the pupil, or a shape parameter of the pupil.

7. A method according to any one of claims 1 to 6 comprising operations of lighting the pupil by means of a light source integrated in the pupillometer, operations of acquiring images of the pupil by a camera of the pupillometer, and operations of processing the images of the pupil as delivered by the camera, enabling the characteristic of the pupil to be determined.

8. A method according to any one of claims 1 to 7, wherein the stimulation is increased in stages, with the total number of stages in a stimulation sequence preferably being less than 100.

9. A method according to any one of claims 1 to 8, wherein the mean rate of increase of the stimulation lies in a range going from approximately 0.1 milliamps per second (mA/s) to approximately 50 mA/s.

10. A method according to any one of claims 1 to 9, wherein the stimuli are pulses forming a signal at a frequency greater than 10 hertz (Hz).

11. A device for controlling stimuli applied to an animal or human subject, the device comprising a pair of electrodes and a pupillometer comprising:
• a stimulation unit connected to the pair of electrodes and comprising:
• a generator unit for generating electrical stimuli, which unit is arranged, and in particular programmed, to generate successive electrical stimuli of intensity increasing between an initial value and a final value, which values are stored in a memory of the pupillometer; and
• a control unit for controlling the application of the electrical stimuli, which unit is arranged, and in particular programmed, to measure the impedance at the terminals of the electrodes;
• a measurement unit arranged, in particular programmed, to act in repeated manner during application of the stimuli to measure at least one size or shape characteristic of the pupil of an eye of the subject; and
• a calculation unit connected to the stimulation unit and to the measurement unit and arranged, in particular programmed, to detect variations of the characteristic measured by the measurement unit and, when a significant variation of the measured characteristic is detected, to cause the increase or the application of the stimuli to be stopped and to store data corresponding to the most recently applied stimuli and, generally, also to at least the most recently measured characteristic;
wherein, in order to detect a significant variation of the measured characteristic of the pupil, the calculation unit compares the variation of the measured characteristic with a predetermined value for variation of this characteristic, which value is stored in a memory of the pupillometer.

12. A device according to claim 11, comprising a camera, a lighting module arranged to light the eye of the subject, a module for acquiring and processing images delivered by the camera and enabling the characteristic of the pupil to be determined, and a communication module connected to the calculation unit so as to enable a user of the device to define measurement, stimulation, and stimulation-control conditions, said device being arranged, in particular programmed, to perform operations of a method according to any one of claims 1 to 10.
